# EUROPEAN PATENT SPECIFICATION

(11) **EP 4 333 726 B1**
(45) Date of publication and mention of the grant of the patent: **07.10.2026**
(21) Application number: 22723201.4
(22) Date of filing: 02.05.2022
(51) Int. Cl.: A61B 17/072, A61B 17/00

(54) **SURGICAL STAPLING DEVICE WITH OPTIMIZED DRIVE ASSEMBLY**
CHIRURGISCHE KLAMMERVORRICHTUNG MIT OPTIMIERTER ANTRIEBSANORDNUNG
DISPOSITIF D'AGRAFAGE CHIRURGICAL AVEC ENSEMBLE D'ENTRAÎNEMENT OPTIMISÉ

(30) Priority: 06.05.2021 US 202117313082
(43) Date of publication of application: 13.03.2024
(73) Proprietor: Covidien LP, Mansfield, MA 02048 (US)
(72) Inventor: BARIL, Jacob C., Norwalk, Connecticut 06851 (US)
(74) Representative: Zimmermann & Partner Patentanwälte mbB
(86) International application number: PCT/IB2022/054035
(87) International publication number: WO 2022/234429

(56) References cited:
- EP-A1- 3 705 055
- US-A1- 2008 169 328
- US-A1- 2019 298 357

## Description

### FIELD

This disclosure is directed to surgical stapling devices, and more particularly, to surgical stapling devices with optimized drive assemblies.

### BACKGROUND

Various types of surgical devices used to endoscopically treat tissue are known in the art, and are commonly used, for example, for closure of tissue or organs in transection, resection, and anastomoses procedures, for occlusion of organs in thoracic and abdominal procedures, and for electrosurgically fusing or sealing tissue.

One example of such a surgical device is a surgical stapling device. Surgical stapling devices include a tool assembly having an anvil assembly, a cartridge assembly, and a drive assembly. Typically, the drive assembly includes a flexible drive beam and a working end that is supported on a distal end of the drive beam. The drive assembly is movable to advance the working end through the tool assembly to approximate the cartridge and anvil assemblies and to advance an actuation sled through the cartridge assembly to eject staples from the cartridge assembly.

Typically, the working end of the drive assembly is formed as a rigid I-beam that is secured to the distal end of the drive beam by welding. When welding is used to secure the rigid I-beam to the flexible drive beam, additional tooling is required to assemble the drive assembly which raises the cost of the manufacturing process. In addition, where the rigid I-beam and the flexible drive beam are improperly welded, the components must be scrapped which also increases the cost of the manufacturing process.

US 2008/169328 A1 discloses an endocutter which comprises a drive bar that is comprised of several layers of material bonded together.

A continuing need exists in the art for a drive assembly that can be manufactured in a more cost-effective manner.

### SUMMARY

This application is directed to a surgical stapling device that includes a drive assembly having a drive member and a working end. The working end of the drive assembly has a I-beam configuration and includes a first beam, a second beam, and a strut interconnecting the first beam and the second beam. The strut defines a slot. The drive member includes a distal portion that is press-fit into the slot in the strut to secure the working end to the drive member.

Aspects of this disclosure are directed to a tool assembly including a first jaw, a second jaw, and a drive assembly. The second jaw is pivotably coupled to the first jaw to facilitate movement of the tool assembly between open and clamped positions. The drive assembly includes a drive member and a working end that defines a slot. The drive member has a distal portion that is press-fit into the slot in the working end to secure the working end to the drive member. The drive assembly is movable between advanced and retracted positions to move the tool assembly between the open and clamped positions.

In aspects of the disclosure, the first jaw includes an anvil, and the second jaw includes a cartridge assembly.

In some aspects of the disclosure, the working end of the drive assembly has an I-beam configuration and includes a first beam, a second beam, and a strut interconnecting the first and second beams.

In certain aspects of the disclosure, the slot is defined in the strut.

In aspects of the disclosure, the cartridge assembly includes a staple cartridge having a cartridge body, staples, pushers, and an actuation sled.

In some aspects of the disclosure, the cartridge body defines staple receiving slots and a central knife slot, and the staple receiving slots are positioned on opposite sides of the knife receiving slot.

In certain aspects of the disclosure, the drive member includes stacked sheets having distal portions, and the distal portions of the stacked sheets and the strut defining openings.

In aspects of the disclosure, the drive assembly includes pins that are received through the openings to secure the drive member to the working end.

In aspects of the disclosure, the strut includes a connector having a circular portion and the drive member includes stacked sheets defining slots that communicate with a circular opening.

In some aspects of the disclosure, the slots in the stacked sheets are aligned with the connector such that upon securement of the working end of the drive assembly to the drive member of the drive assembly, the circular portion of the connector is received within the circular openings of the stacked sheets.

In certain aspects of the disclosure, the drive member includes stacked sheets having distal portions that define a hook and a slot, and the strut includes a connector positioned within the slot of the working end of the drive assembly such that the connector is received within the slots of the stacked sheets when the working end is secured to the drive member.

In aspects of the disclosure, the drive member includes stacked sheets having distal portions with outwardly flared ends that are press-fit into the slot in the working end of the drive assembly.

In some aspects of the disclosure, the slot in the working end of the drive assembly includes outwardly flared portions that receive the outwardly flared ends of the stacked sheets of the drive member.

In certain aspects of the disclosure, the cartridge assembly and the anvil are pivotable in relation to each other between the open and clamped positions.

In aspects of the disclosure, the first beam of the drive assembly is engaged with the anvil and the second beam of the drive assembly is engaged with the cartridge assembly.

In some aspects of the disclosure, the distal portion of the drive member of the drive assembly supports a cylindrical member and the slot in the working end of the drive assembly has a cylindrical portion.

In certain aspects of the disclosure, the cylindrical member is press-fit into the cylindrical portion of the slot in the working end of the drive assembly.

Other aspects of the disclosure are directed to a surgical device including an adapter assembly and a tool assembly. The adapter assembly has a proximal portion and a distal portion, and the tool assembly is coupled to the distal portion of the adapter assembly and includes a first jaw, a second jaw, and a drive assembly. The second jaw is pivotably coupled to the first jaw to facilitate movement of the tool assembly between open and clamped positions. The drive assembly includes a drive member and a working end having an I-beam configuration including a first beam, a second beam, and a strut interconnecting the first and second beams and defining a slot. The drive member has a distal portion that is press-fit into the slot to secure the working end to the drive member such that the drive assembly is movable between advanced and retracted positions to move the tool assembly between the open and clamped positions.

Still other aspects of the disclosure are directed to a surgical stapling device including an adapter assembly and a tool assembly. The adapter assembly has a proximal portion and a distal portion, and the tool assembly is pivotably coupled to the distal portion of the adapter assembly. The tool assembly includes an anvil, a cartridge assembly, and a drive assembly. The cartridge assembly is pivotably coupled to the anvil to facilitate movement of the tool assembly between open and clamped positions and includes a channel member and a staple cartridge. The staple cartridge includes a cartridge body, staples, pushers, and an actuation sled. The cartridge body defines staple receiving slots and a central knife slot. The staple receiving slots are positioned on opposite sides of the knife receiving slot and receives the staples and the pushers. The drive assembly includes a drive member, and a working end having an I-beam configuration that includes a first beam, a second beam, and a strut interconnecting the first and second beams. The strut defines a slot. The drive member has a distal portion that is press-fit into the slot to secure the working end to the drive member such that the drive assembly is movable between advanced and retracted positions to move the tool assembly between the open and clamped positions.

The invention is defined by the appended claims.

Other features of the disclosure will be appreciated from the following description.

### BRIEF DESCRIPTION OF THE DRAWINGS

Various aspects of the disclosed surgical stapling device are described herein below with reference to the drawings, wherein:
FIG. 1 is a side perspective view of a surgical stapling device according to aspects of the disclosure with a tool assembly of the stapling device in an open position;
FIG. 2 is an enlarged view of the indicated area of detail shown in FIG. 1;
FIG. 3 is a side perspective, exploded view of the tool assembly of the surgical stapling device shown in FIG. 1;
FIG. 4 is a drive assembly of the tool assembly of the surgical stapling device shown in FIG. 1;
FIG. 5 is a side perspective, exploded view of the drive assembly of the tool assembly shown in FIG. 4;
FIG. 6 is an alternate version of the drive assembly of the surgical stapling device shown in FIG. 1;
FIG. 7 side perspective, exploded view of the drive assembly shown in FIG. 6;
FIG. 8 is a cross-sectional view taken along section line 8-8 of FIG. 6;
FIG. 9 is another alternate version of the drive assembly of the surgical stapling device shown in FIG. 1;
FIG. 10 side perspective, exploded view of the drive assembly shown in FIG. 9;
FIG. 11 is a cross-sectional view taken along section line 11-11 of FIG. 9;
FIG. 12 is a side perspective, cutaway view of an embodiment of the drive assembly of the surgical stapling device shown in FIG. 1, in accordance with the present invention;
FIG. 13 is a side perspective view of the distal portion of the drive assembly shown in FIG. 13 assembled;
FIG. 14 is a side perspective, cutaway view of yet another alternate version of the drive assembly of the surgical stapling device shown in FIG. 1; and
FIG. 15 is a side perspective view of the distal portion of the drive assembly shown in FIG. 14 assembled.

### DETAILED DESCRIPTION

The disclosed surgical stapling device will now be described in detail with reference to the drawings in which like reference numerals designate identical or corresponding elements in each of the several views. However, it is to be understood that aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

As used herein, the term "distal" refers to the portion of the stapling device that is being described which is further from a user during use of the device in its customary manner, while the term "proximal" refers to the portion of the stapling device that is being described which is closer to a user during use of the device in its customary manner. In addition, directional terms such as front, rear, upper, lower, top, bottom, and similar terms are used to assist in understanding the description and are not intended to limit the disclosure.

This application is directed to a surgical stapling device that includes a tool assembly and an adapter assembly. The tool assembly includes a drive assembly having a drive member and a working end. The working end of the drive assembly has an I-beam configuration and includes a first beam, a second beam, and a strut interconnecting the first beam and the second beam. The strut defines a slot. The drive member includes a distal portion that is press-fit into the slot in the strut to secure the working end to the drive member.

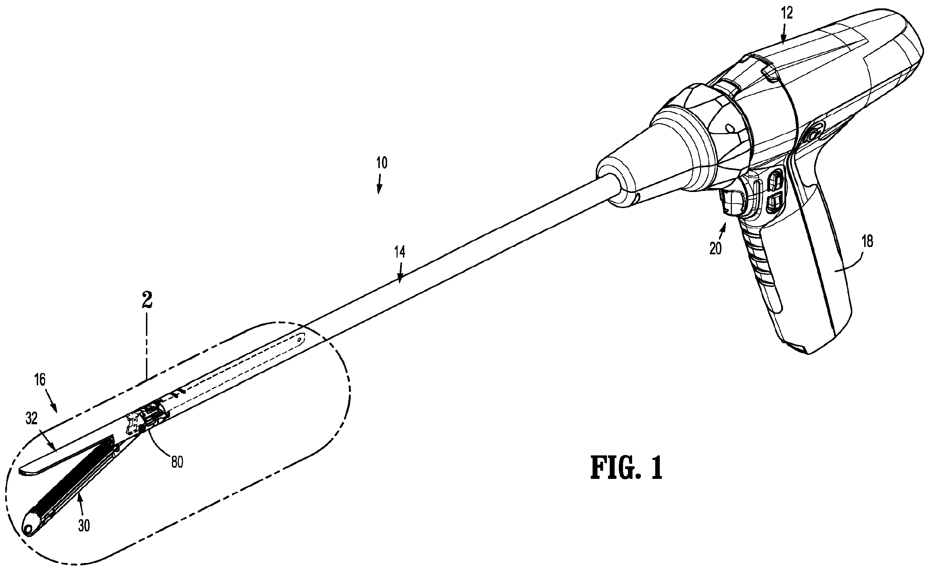

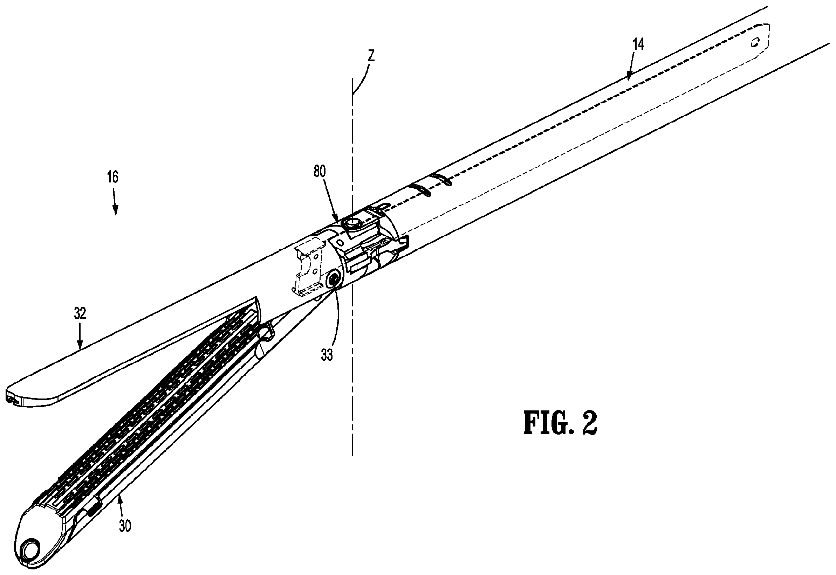

FIGS. 1 and 2 illustrate a surgical stapling device shown generally as stapling device 10 that includes a handle assembly 12, an elongate body or adapter assembly 14, and a tool assembly 16. As illustrated, the handle assembly 12 is powered and includes a stationary handgrip 18 and actuation buttons 20. The actuation buttons 20 are operable to actuate various functions of the tool assembly 16 via the adapter assembly 14 including approximation, stapling, cutting, and articulation. In certain aspects of the disclosure, the handle assembly 16 supports batteries (not shown) that provide power to the handle assembly 12 to operate the stapling device 10. Although the stapling device 10 is illustrated as a powered stapling device, it is envisioned that the features of this disclosure are suitable for use with manually powered surgical stapling devices as well as robotically controlled stapling devices. It is also envisioned that aspects of this disclosure are suitable for use with surgical devices, other than stapling devices, that include a drive assembly having a flexible drive beam and an I-beam secured to the flexible drive beam.

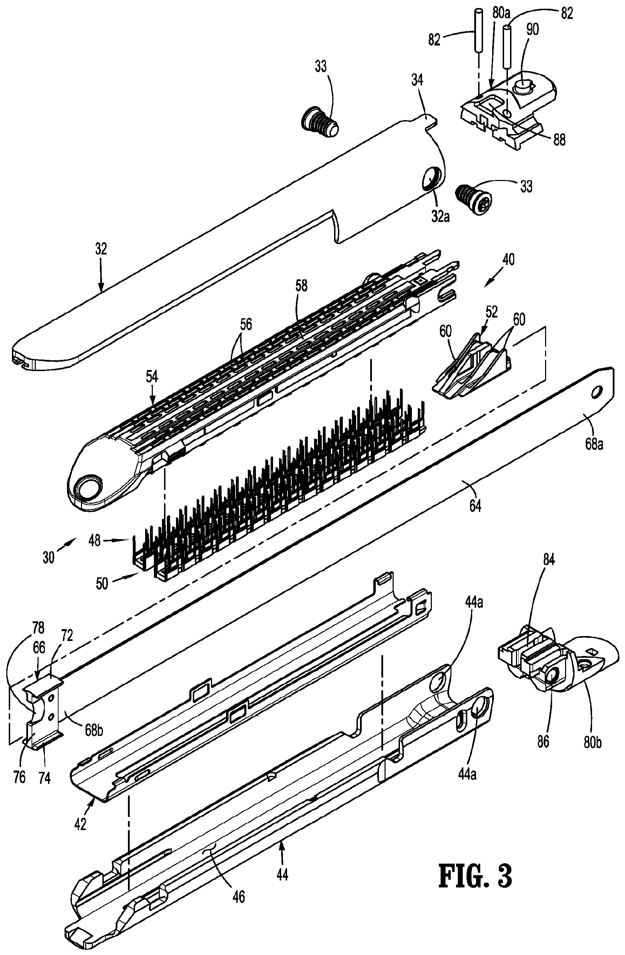

FIGS. 2 and 3 illustrate the tool assembly 16 of the stapling device 10 which includes a cartridge assembly 30 and an anvil 32 that are pivotably coupled together by screws or pins 33. The anvil 32 defines openings 32a that receive the screws 33 and includes a proximally extending tab 34. The cartridge assembly 30 and the anvil 32 are coupled together such that the tool assembly 16 can pivot between open and clamped positions. The cartridge assembly 30 includes a staple cartridge 40, a staple guard 42, and a channel member 44. The channel member 44 defines a cavity 46 that receives the staple cartridge 40 and openings 44a that receive the screws 33. In aspects of the disclosure, the staple cartridge 40 can be releasable from the cavity 46 of the channel member 44 to facilitate replacement of the staple cartridge 40 from the channel member 44 and reuse the stapling device 10. Alternately, the staple cartridge 40 can be fixedly received within the cavity 46 of the channel member 44.

The staple cartridge 40 includes staples 48, pushers 50, an actuation sled 52, and a cartridge body 54. The cartridge body 54 defines a plurality of staple receiving slots 56 and a central knife slot 58. In aspects of the disclosure, the staple receiving slots 56 are aligned in rows on opposite sides of the central knife slot 58 although other arrays of staple receiving slots 56 are envisioned. Each of the staple receiving slots 56 receives one of the staples 48 and one of the pushers 50. The actuation sled 52 includes ramps 60 that are movable through the cartridge body 54 into engagement with the pushers 50 to eject the staples 48 from the cartridge body 54. The staple guard 42 is secured to a bottom of the cartridge body 54 to retain the staples 48 and the pushers 50 within the cartridge body 54. For a more detailed description of the construction and operation of an exemplary cartridge assembly, see U.S. Patent No. 7,891,534 (the '534 patent).

FIGS. 3-5 illustrate the drive assembly 38 of the stapling device 10 (FIG. 1) which includes a drive member 64 and a working end 66. In aspects of the disclosure, the drive member 64 includes a proximal portion 68a and a distal portion 68b. The proximal portion 68a is coupled to a drive mechanism (not shown) within the adapter assembly 14 of the stapling device 10 (FIG. 1) such that longitudinal movement of the drive mechanism causes longitudinal movement of the drive assembly 38. The distal portion 68b of the drive member 64 is secured to the working end 66 of the drive assembly 38 such that longitudinal movement of the drive member 64 causes longitudinal movement of the working end 66. In some aspects of the disclosure, the drive member 64 is formed from flexible metal sheets, e.g., steel.

In aspects of the disclosure, the working end 66 of the drive assembly 38 is rigid and has an I-beam configuration that includes a first beam 72, a second beam 74, and a vertical strut 76. The first beam 72 is supported on one end of the vertical strut 76 and the second beam 74 is supported on an opposite end of the vertical strut 76. In aspects of the disclosure, the vertical strut 76 includes or supports a cutting blade 78. The first beam 72 is engaged with the anvil 52 and the second beam 74 is engaged with the channel member 44 such that longitudinal movement of the working end 66 of the drive assembly 38 causes the tool assembly 16 to move between the open and clamped positions. The vertical strut 76 of the working end 68 of the drive assembly 38 is positioned proximally of and in alignment with the actuation sled 52 such that advancement of the working end 68 of the drive assembly 38 in relation to the anvil 32 and the cartridge assembly 30 causes advancement of the actuation sled 52 within the cartridge assembly 30 to eject the staples from the cartridge body 54. In aspects of the disclosure, the working end 68 of the drive assembly 38 is formed from metal, e.g., stainless steel, although other materials of construction are envisioned.

The tool assembly 16 includes a coupling assembly 80 (FIG. 2) having a first coupling member 80a (FIG. 3) and a second coupling member 80b which are secured together by pins 82. The second coupling member 80b defines a longitudinal channel 84 that receives the drive member 64 and side openings 86 that receive the screws 33 (FIG. 3). The screws 33 extend through the openings 44a in the channel member 44, through the openings 32a in the anvil 32, and into the side openings 86 in the second coupling member 80b to secure the channel member 44 of the cartridge assembly 30 and the anvil 30 to the coupling assembly 80. The first coupling member 80a includes a cutout 88 (FIG. 3) that receives the tab 34 of the anvil 32. When the first coupling member 80a is coupled to the second coupling member 80b with the pins 82, the first coupling member 80a encloses the channel 88 in the second coupling member 80b and the coupling assembly 80 is secured to the proximal portion of the anvil 32 and cartridge assembly 30. In an assembled condition, the cartridge assembly 30 can pivot in relation to the anvil 32 about the screws 33 (FIG. 3) between the open and clamped positions while the anvil 32 is fixed in relation to the coupling assembly 80. It is envisioned that the cartridge assembly 30 could be fixedly mounted to the mounting assembly 80 while the anvil 32 could pivot between the open and clamped positions.

Each of the first and second coupling members 80a and 80b includes a pivot member 90 (only one is shown) that define an articulation axis Z (FIG. 2). The pivot members 90 are coupled to the distal portion of the adapter assembly 14 (FIG. 1) to pivotably secure the tool assembly 16 to the adapter assembly 14 to facilitate articulation of the tool assembly 16 about the articulation axis "Z". For a more detailed description of the pivotable connection between the tool assembly 16 and the adapter assembly 14, see the '534 Patent.

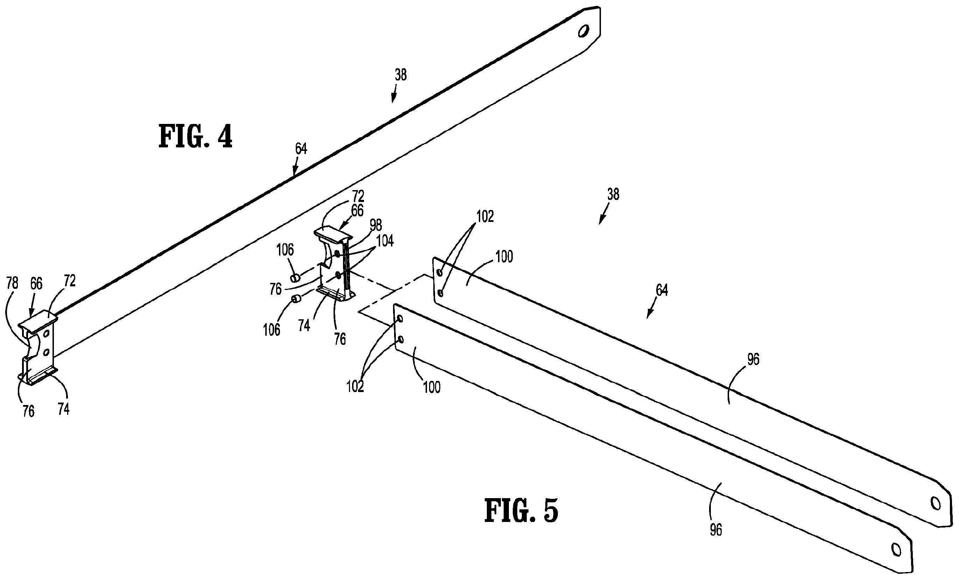

In aspects of the disclosure, the drive member 64 of the drive assembly 38 is formed from stacked sheets 96 (FIG. 5) and the working end 66 of the drive assembly 38 defines a slot 98. The slot 98 is formed in the vertical strut 76 of the working end 66 of the drive assembly 38 and has an open proximal end. The stacked sheets 96 have distal portions 100 that are received within the open proximal end of the slot 98. In some aspects of the disclosure, the distal portions 100 of the stacked sheets 96 are press fit into the slot 98 to secure the drive member 64 to the working end 66 of the drive assembly 38. In certain aspects of the disclosure, the distal portions 100 of the stacked sheets 96 define openings 102 and the vertical strut 76 of the working end 66 of the drive assembly 38 defines openings 104. The openings 102 and 104 receive pins 106 that are press fit into the openings 102 and 104 to secure the working end 66 to the stacked sheets 96 of the drive member 64.

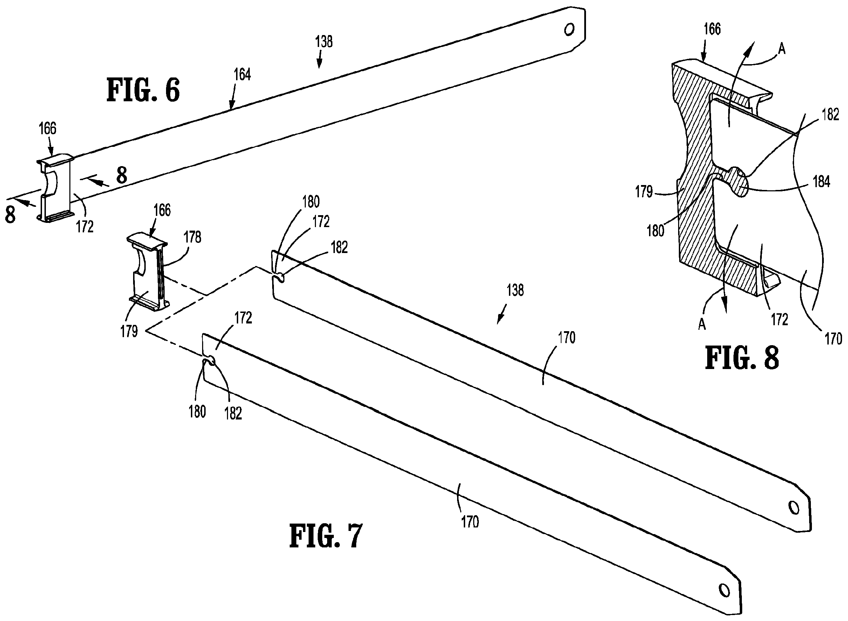

FIGS. 6-8 illustrate an alternative version of the drive assembly of the stapling device 10 (FIG. 1) shown generally as drive assembly 138. The drive assembly 138 is substantially similar to the drive assembly 38 (FIG. 4) except for the connection between the drive member 164 and the working end 166 of the drive assembly 138. The drive member 164 of the drive assembly 138 includes stacked sheets 170 that have distal portions 172, and the drive member 164 defines a slot 178 that is formed in the vertical strut 179 of the working end 166. The distal portions 172 of the stacked sheets 170 define slots 180 that extend through the distal ends of the stacked sheets 170 and communicate with circular bores 182 that have diameters that are greater than the widths of the slots 180. The vertical strut 179 includes a connector 184 that is positioned within the slot 178 and is aligned with the slots 180. In aspects of the disclosure, the connector 184 has a circular proximal portion that has a diameter that corresponds to the diameter of the circular bores 182 in the stacked sheets 170.

When the distal portions 172 of the of the stacked sheets 170 are inserted into the slot 178 of the vertical strut 179, the connector 184 in the vertical strut 179 of the working end 166 of the drive assembly 138 is received in the slots 180 of the stacked sheets 170. When the connector 182 is received in the slots 180 of the stacked sheets 170, the distal portions 172 of the stacked sheets 170 flex outwardly in the direction of arrows "A" in FIG. 8 to allow the circular proximal portion of the connector 184 to be received within the circular bores 182 of the stacked sheets 170. In this respect, the height of the slot 178 in the vertical strut 179 is larger than the height of the stacked sheets 170 to allow for outward deformation of the distal portions 172 of the stacked sheets 170. When the circular distal portion of the connector 184 is received within the circular bores 182 of the stacked sheets 170, the distal portions 172 of the stacked sheets return to their original undeformed configuration to help secure the stacked sheets 170 of the drive member 164 to the working end 166 of the drive assembly 138. In aspects of the disclosure, the distal portions 172 of the stacked sheets 170 have a width that is substantially equal to the width of the slot 178 in the vertical strut 179 and the stacked sheets are press-fit into the slot 178 in the vertical strut 179.

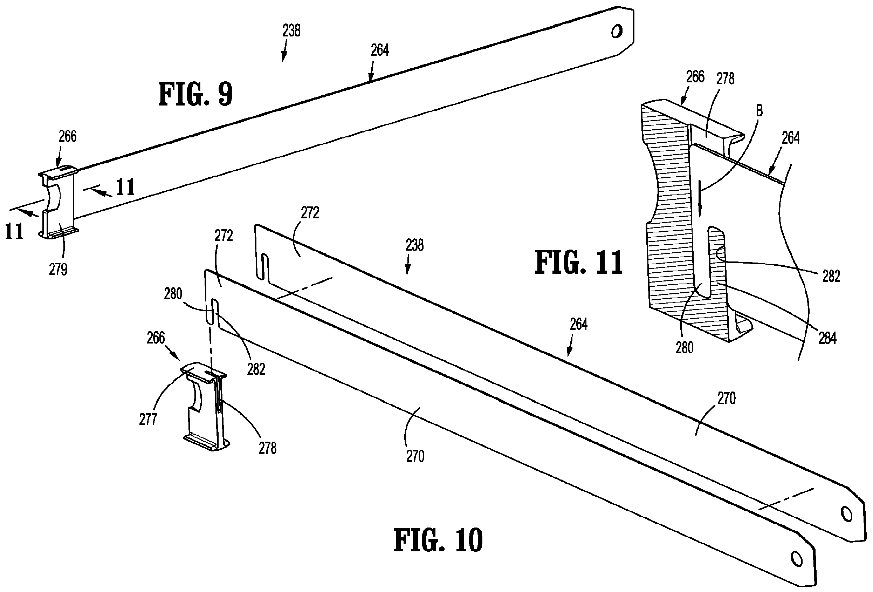

FIGS. 9-11 illustrate another alternative version of the drive assembly of the stapling device 10 (FIG. 1) shown generally as drive assembly 238. The drive assembly 238 is substantially similar to the drive assemblies 38 (FIG. 4) and 138 (FIG. 7) except for the connection between the drive member 264 and the working end 266 of the drive assembly 238. The drive member 264 of the drive assembly 238 includes stacked sheets 270 that have distal portions 272, and the drive member 264 defines a slot 278 that is formed through the first beam 277 and the vertical strut 279 of the working end 266 of the drive assembly 238. The distal portions 272 of the stacked sheets 270 define hooks 280 and slots 282. The vertical strut 279 of the working end 266 of the drive member 238 includes a connector 284 that is in the form of a hook that is positioned in the slot 278. The slot 278 in the vertical strut 279 has an open upper end.

When the drive member 264 is coupled to the working end 266 of the drive assembly 238, the distal portions of the stacked sheets 270 are press-fit into the slot 278 in the vertical strut 279 in the direction indicated by arrow "B" in FIG. 11 such that the hooks 280 of the stacked sheets 270 are received distally of the connector 284 of the vertical strut 279 and the connector 284 is received in the slots 282 of the stacked sheets 270 to secure the drive member 264 to the working end 266 of the drive assembly 238.

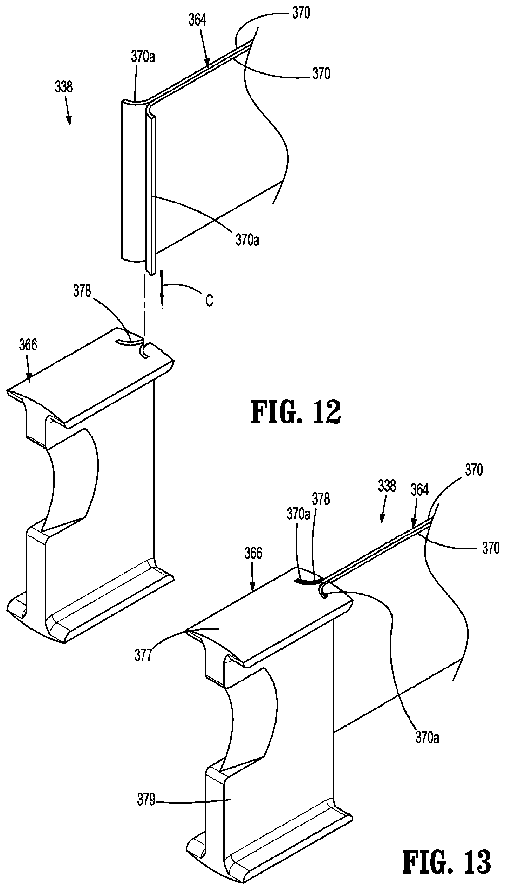

FIGS. 12 and 13 illustrate a distal portion of a drive assembly of the stapling device 10 (FIG. 1) shown generally as drive assembly 338, which is in accordance with the present invention. The drive assembly 338 is substantially similar to the drive assemblies 38 (FIG. 4), 138 (FIG. 7), and 238 (FIG. 9) except for the connection between the drive member 364 and the working end 366 of the drive assembly 338. In the drive assembly 338, the drive member 364 includes stacked sheets 370 that have outwardly flared ends 370a, and the working end 366 of the drive assembly 338 defines a slot 378 including outwardly flared portions 378a that receive the outwardly flared ends 370a of the stacked sheets 370. The slot 378 extends through the first beam 377 and vertical strut 379 of the working end 366 of the drive assembly 338. When the stacked sheets 370 are secured to the working end 366 of the drive assembly 338, the flared ends 370a of the stacked sheets 370 of the drive assembly 338 are press fit into the flared portions 378a of the slot 378 in the direction indicated by arrow "C" in FIG. 12 to securely fasten the drive member 364 to the working end 366 of the drive assembly 338.

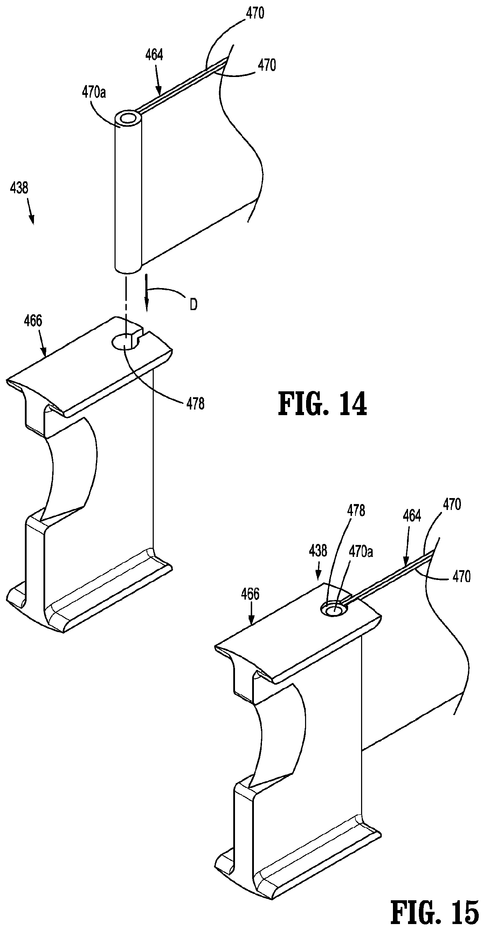

FIGS. 14 and 15 illustrate a distal portion of yet another alternative version of the drive assembly of the stapling device 10 (FIG. 1) shown generally as drive assembly 438. The drive assembly 438 is substantially similar to the drive assemblies 38 (FIG. 4), 138 (FIG. 7), 238 (FIG. 9), and 338 except for the connection between the drive member 464 and the working end 466 of the drive assembly 438. In the drive assembly 438, the drive member 464 includes stacked sheets 470 that have a cylindrical member 470a secured to or integrally formed with the stacked sheets 470, and the working end 466 of the drive assembly 438 defines a cylindrical slot 478 that receives the cylindrical member 470a of the drive member 464. The slot 478 extends through the first beam 477 and the vertical strut 479 of the working end 466 of the drive assembly 438. When the stacked sheets 470 are secured to the working end 466 of the drive assembly 438, the cylindrical member 470a of the stacked sheets 470 of the drive assembly 438 is press fit into the cylindrical slot 478 of the working end 466 of the drive assembly 438 in the direction indicated by arrow "D" in FIG. 12 to securely fasten the drive member 464 to the working end 466 of the drive assembly 438.

Each of the drive assemblies described above includes a drive member including stacked sheets that have distal portions that are press-fit into a slot defined in a working end of the drive assembly to fixedly secure the drive member to the working end. Press-fitting the drive member onto the working end of the drive assembly obviates the need for welding the components of the drive assembly to each other to simplify and reduce costs associated with the manufacturing process.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. A tool assembly (16) comprising:
a first jaw;
a second jaw pivotably coupled to the first jaw to facilitate movement of the tool assembly between open and clamped positions; and
a drive assembly (38, 138, 238, 338, 438) including a drive member (64, 164, 264, 364, 464) and a working end (66, 166, 266, 366, 466), the working end defining a slot (98, 178, 278, 378, 478),
the drive member having a distal portion (68b) that is press-fit into the slot to secure the working end to the drive member, wherein the drive assembly is movable between advanced and retracted positions to move the tool assembly between the open and clamped positions;
wherein the first jaw includes an anvil (32), and the second jaw includes a cartridge assembly (30),
wherein the working end of the drive assembly has an I-beam configuration and includes a first beam (72, 277, 377, 477), a second beam (74), and a strut (76, 179, 279, 379, 479)
interconnecting the first and second beams, the slot defined in the strut; wherein the drive member includes stacked sheets (96, 170, 270, 370, 470) **characterised in that** each of the stacked sheets including a distal portion (100, 172, 272) having an outwardly flared end (370a) that is press-fit into the slot in the working end of the drive assembly; wherein the slot in the working end of the drive assembly includes outwardly flared portions (378a) that receive the outwardly flared ends of the stacked sheets of the drive member.

2. The tool assembly of claim 1, wherein the cartridge assembly includes a staple cartridge (40), the staple cartridge including a cartridge body(54), staples (48), pushers (50), and an actuation sled (52), the cartridge body defining staple receiving slots (56) and a central knife slot (58),
the staple receiving slots
positioned on opposite sides of the knife receiving slot.

3. The tool assembly of claim 1, wherein the cartridge assembly and the anvil are pivotable in relation to each other between the open and clamped positions.

4. The tool assembly of claim 1, wherein the first beam of the drive assembly is engaged with the anvil and the second beam of the drive assembly is engaged with the cartridge assembly.

5. A surgical device (10) comprising:
an adapter assembly (14) having a proximal portion and a distal portion; and
a tool assembly (16)
coupled to the distal portion of the adapter assembly, the tool assembly as claimed in claims 1 to 4.

## Patentansprüche

1. Werkzeugbaugruppe (16), umfassend:
eine erste Backe,
eine zweite Backe, die schwenkbar an die erste Backe gekoppelt ist, um eine Bewegung der Werkzeugbaugruppe zwischen einer offenen und einer geklemmten Position zu ermöglichen, und
eine Antriebsbaugruppe (38, 138, 238, 338, 438), die ein Antriebsglied (64, 164, 264, 364, 464) und ein Arbeitsende (66, 166, 266, 366, 466) aufweist, wobei das Arbeitsende einen Schlitz (98, 178, 278, 378, 478) definiert, wobei das Antriebsglied einen distalen Abschnitt (68b) aufweist, der in den Schlitz eingepresst ist, um das Arbeitsende an dem Antriebsglied zu befestigen, wobei die Antriebsbaugruppe zwischen vorgeschobenen und zurückgezogenen Positionen beweglich ist, um die Werkzeugbaugruppe zwischen der offenen und der geklemmten Position zu bewegen,
wobei die erste Backe einen Amboss (32) aufweist und die zweite Backe eine Magazinbaugruppe (30) aufweist,
wobei das Arbeitsende der Antriebsbaugruppe eine I-Träger-Konfiguration aufweist und einen ersten Träger (72, 277, 377, 477), einen zweiten Träger (74) und eine Strebe (76, 179, 279, 379, 479) aufweist, die den ersten und den zweiten Träger miteinander verbindet, wobei der Schlitz in der Strebe definiert ist, wobei das Antriebsglied gestapelte Bleche (96, 170, 270, 370, 470) aufweist, **dadurch gekennzeichnet, dass** jedes der gestapelten Bleche einen distalen Abschnitt (100, 172, 272) aufweist, der ein nach außen aufgeweitetes Ende (370a) aufweist, das in den Schlitz in der Arbeitsbaugruppe eingepresst ist, wobei der Schlitz in dem Arbeitsende der Antriebsbaugruppe nach außen aufgeweitete Abschnitte (378a) aufweist, die die nach außen aufgeweitete Enden der gestapelten Bleche des Antriebsglieds aufnehmen.

2. Werkzeugbaugruppe nach Anspruch 1, wobei die Magazinbaugruppe ein Klammermagazin (40) aufweist, wobei das Klammermagazin einen Magazinkörper (54), Klammern (48), Schieber (50) und einen Betätigungsschlitten (52) aufweist, wobei der Magazinkörper Klammeraufnahmeschlitze (56) und einen mittleren Messerschlitz (58) definiert, wobei die Klammeraufnahmeschlitze auf gegenüberliegenden Seiten des Messeraufnahmeschlitzes positioniert sind.

3. Werkzeugbaugruppe nach Anspruch 1, wobei die Magazinbaugruppe und der Amboss bezüglich einander zwischen der offenen und der geklemmten Position schwenkbar sind.

4. Werkzeugbaugruppe nach Anspruch 1, wobei der erste Träger der Antriebsbaugruppe mit dem Amboss in Eingriff steht und der zweite Träger der Antriebsbaugruppe mit der Magazinbaugruppe in Eingriff steht.

5. Chirurgische Vorrichtung (10), umfassend:
eine Adapterbaugruppe (14) mit einem proximalen Abschnitt und einem distalen Abschnitt und
eine an den distalen Abschnitt der Adapterbaugruppe gekoppelte Werkzeugbaugruppe (16), wobei die Werkzeugbaugruppe wie in Ansprüchen 1 bis 4 beansprucht ist.

## Revendications

1. Ensemble d'outils (16) comprenant :
une première mâchoire ;
une seconde mâchoire accouplée pivotante à la première mâchoire pour faciliter le mouvement de l'ensemble d'outils entre des positions ouverte et serrée ; et
un ensemble d'entraînement (38, 138, 238, 338, 438) comportant un élément d'entraînement (64, 164, 264, 364, 464) et une extrémité de travail (66, 166, 266, 366, 466), l'extrémité de travail définissant une fente (98, 178, 278, 378, 478), l'élément d'entraînement ayant une partie distale (68b) qui est ajustée à force dans la fente pour fixer l'extrémité de travail à l'élément d'entraînement, l'ensemble d'entraînement étant mobile entre des positions avancée et rétractée pour déplacer l'ensemble d'outils entre les positions ouverte et serrée ;
dans lequel la première mâchoire comporte une enclume (32), et la seconde mâchoire comporte un ensemble cartouche (30),
dans lequel l'extrémité de travail de l'ensemble d'entraînement a une configuration en I et comporte une première barre (72, 277, 377, 477), une seconde barre (74), et une entretoise (76, 179, 279, 379, 479) interconnectant les première et seconde barres, la fente étant définie dans l'entretoise ; dans lequel l'élément d'entraînement comporte des feuilles empilées (96, 170, 270, 370, 470), **caractérisé en ce que** chacune des feuilles empilées comporte une partie distale (100, 172, 272) ayant une extrémité évasée vers l'extérieur (370a) qui est ajustée à force dans la fente dans l'extrémité de travail de l'ensemble d'entraînement ; dans lequel la fente dans l'extrémité de travail de l'ensemble d'entraînement comporte des parties évasées vers l'extérieur (378a) qui reçoivent les extrémités évasées vers l'extérieur des feuilles empilées de l'élément d'entraînement.

2. Ensemble d'outils selon la revendication 1, dans lequel l'ensemble cartouche comporte une cartouche d'agrafes (40), la cartouche d'agrafes comportant un corps de cartouche (54), des agrafes (48), des poussoirs (50) et un chariot d'actionnement (52), le corps de cartouche définissant des fentes de réception d'agrafes (56) et une fente de couteau centrale (58), les fentes de réception d'agrafes étant positionnées sur des côtés opposés de la fente de réception de couteau.

3. Ensemble d'outils selon la revendication 1, dans lequel l'ensemble cartouche et l'enclume peuvent pivoter l'un par rapport à l'autre entre les positions ouverte et serrée.

4. Ensemble d'outils selon la revendication 1, dans lequel la première barre de l'ensemble d'entraînement est en prise avec l'enclume et la seconde barre de l'ensemble d'entraînement est en prise avec l'ensemble cartouche.

5. Dispositif chirurgical (10) comprenant :
un ensemble adaptateur (14) ayant une partie proximale et une partie distale ; et
un ensemble d'outils (16) accouplé à la partie distale de l'ensemble adaptateur, l'ensemble d'outils étant selon les revendications 1 à 4.
